# EUROPEAN PATENT APPLICATION

(11) **EP 3 138 560 A1**
(43) Date of publication of application: **08.03.2017**
(21) Application number: 15786060.2
(22) Date of filing: 27.04.2015
(51) Int. Cl.: A61K 31/13, A61P 27/16

(54) **DRUG FOR TREATMENT OF TINNITUS PATIENTS**

(30) Priority: 28.04.2014 JP 2014092873
(71) Applicant: Kyorin Pharmaceutical Co., Ltd., Tokyo 101-8311 (JP)
(72) Inventor: DOI, Katsumi, Osakasayama-shi Osaka 589-8511 (JP); HIRAHARA, Yoshifumi, Tokyo 101-8311 (JP); NAGAI, Shinji, Tokyo 101-8311 (JP); USHIWATA, Ai, Tokyo 101-8311 (JP); NIWAYAMA, Ikuyo, Tokyo 101-8311 (JP); WATANABE, Koichi, Tokyo 101-8311 (JP); TOMINAGA, Madoka, Tokyo 101-8311 (JP); KUROSE, Takafumi, Tokyo 101-8311 (JP); YAMADA, Shinichi, Tokyo 101-8311 (JP); SHINOZAKI, Takehiro, Tokyo 101-8311 (JP); CSIKOS, Janos, 60438 Frankfurt am Main (DE); GORTELMEYER, Roman, 60438 Frankfurt am Main (DE)
(74) Representative: Ricker, Mathias
(86) International application number: PCT/JP2015/062713
(87) International publication number: WO 2015/166918

(57) **Abstract**

A drug for treatment of patients having tinnitus associated with age-related hearing loss, comprising neramexane or a pharmaceutically acceptable salt thereof.

## Description

### [Technical Field]

The present invention relates to a drug for treatment of tinnitus patients.

### [Background Art]

Inner ear damage is a major problem in modern society. Tinnitus, in association with hearing loss, is a common condition encountered with inner ear damage. Tinnitus is defined as either "reception of sound among auditory stimulation lacking from the external environment" or "an abnormal auditory phenomenon produced by sensation of a sound, despite the lack of a sound source outside of the body". Tinnitus can be produced by damage to any part of the auditive pathway from the external ear to the central auditive pathway, but it is generally produced by inner ear hearing loss, and the condition of tinnitus is characterized by increased abnormal nerve activity in the central auditive pathway due to reduced input of auditory information resulting from cochlear damage. For the individual patient, tinnitus is sometimes tolerable but in some cases can lead to anxiety, depression, insomnia, disability or mental distress.

While many factors are involved in tinnitus, it has been disclosed, in PTL 1 for example, that 1-aminoalkylcyclohexane derivatives (especially neramexane) and their pharmaceutically acceptable salts are effective for treatment of patients suffering from cochlear tinnitus.

### [Citation List]

### [Patent Literature]

[PTL 1] Japanese Patent Public Inspection No. 2010-539118

### [Summary of the Invention]

### [Problems to be Solved by the Invention]

Incidentally, PTL 1 does not sufficiently investigate the possibility that more effective treatment can be carried out if the patient group is a group with a potentially greater drug effect among patients suffering from cochlear tinnitus.

It is therefore an object of the present invention to provide a drug for treatment of tinnitus patients that allows more effective treatment to be carried out specifically for a patient group expected to experience a greater drug effect.

### [Means for Solving the Problems]

Specifically, the invention provides the following [1] to [9].
[1] A drug for treatment of patients having tinnitus associated with age-related hearing loss, comprising neramexane or a pharmaceutically acceptable salt thereof.
[2] A drug for treatment of patients having tinnitus associated with auditory disorder in the range of the human voice at an auditory threshold value exceeding 25 dB, comprising neramexane or a pharmaceutically acceptable salt thereof.
[3] A drug for treatment of patients having tinnitus associated with age-related hearing loss, comprising neramexane or a pharmaceutically acceptable salt thereof, wherein the age-related hearing loss exhibits auditory disorder in the range of the human voice at an auditory threshold value exceeding 25 dB.
[4] A drug for treatment of patients having tinnitus, comprising neramexane or a pharmaceutically acceptable salt thereof, wherein the tinnitus includes two or more different tinnitus sounds.
[5] A drug for treatment of patients having tinnitus associated with age-related hearing loss, comprising neramexane or a pharmaceutically acceptable salt thereof, wherein the tinnitus includes two or more different tinnitus sounds.
[6] A drug for treatment of patients having tinnitus associated with age-related hearing loss, comprising neramexane or a pharmaceutically acceptable salt thereof, wherein the age-related hearing loss exhibits auditory disorder in the range of the human voice at an auditory threshold value exceeding 25 dB, and wherein the tinnitus includes two or more different tinnitus sounds.
[7] A drug for treatment of patients having tinnitus, comprising neramexane or a pharmaceutically acceptable salt thereof, wherein the tinnitus sound is not high-frequency alone.
[8] A drug for treatment of patients having tinnitus associated with age-related hearing loss, comprising neramexane or a pharmaceutically acceptable salt thereof, wherein the age-related hearing loss is at hearing threshold of 25dB or more in both ears in 8000Hz.
[9] A drug for treatment of patients having tinnitus associated with age-related hearing loss, comprising neramexane or a pharmaceutically acceptable salt thereof, wherein the age-related hearing loss is at hearing threshold of 25dB or more in both ears in 4000Hz and 8000Hz.

### [Effect of the Invention]

According to the invention it is possible to provide a drug for treatment of tinnitus patients that allows more effective treatment to be carried out specifically for a patient group expected to experience a greater drug effect.

### [Mode for Carrying Out the Invention]

The terms and symbols used herein will now be explained with a more detailed description of the invention, with the understanding that the invention is not limited thereto.

Neramexane (chemical name: 1-amino-1,3,3,5,5-pentamethylcyclohexane) is disclosed in US Patent No. 6,034,134 and No. 6,071,966, for example. This compound has been found to be useful for treatment of various diseases and particularly for certain neuropathies. The therapeutic effect of neramexane is putatively due to inhibition of the effect of excess glutamic acid at neuronic N-methyl-D-aspartic acid (NMDA) receptors. For this reason, the compound is classified as an NMDA receptor antagonist. In addition, it has been disclosed that neramexane exhibits inhibiting activity on α9/α10 nicotinic acetylcholine receptors (Plazas, et al., Eur J Pharmacol., 2007 Jul. 2; 566(1-3):11-19) and 5-HT₃ receptors.

For the purpose of this embodiment, neramexane may be used in any form including as a pharmaceutically acceptable salt, solvate compound, isomer, conjugant, prodrug, polymorphism, derivative or a mixture of the foregoing. The term "neramexane" as used herein, unless otherwise specified, is to be interpreted as also referring to a pharmaceutically acceptable salt, solvate compound, conjugant, prodrug, polymorphism or derivative thereof, or a mixture of the foregoing.

For the purpose of the present specification, the term "pharmaceutically acceptable salt" means a salt of 1-amino-1,3,3,5,5-pentamethylcyclohexane that can be obtained by combination with an inorganic or organic acid, and that does not affect human safety and/or that is sufficiently accepted by humans after administration. Examples of pharmaceutically acceptable salts include, but are not limited to, acid addition salts such as salts produced from hydrochloric acid, hydrobromic acid, hydroiodic acid, perchloric acid, sulfuric acid, nitric acid, phosphoric acid, acetic acid, propionic acid, glycolic acid, lactic acid, pyruvic acid, malonic acid, succinic acid, fumaric acid, tartaric acid, citric acid, benzoic acid, carbonic acid, cinnamic acid, mandelic acid, methanesulfonic acid (mesylic acid), ethanesulfonic acid, hydroxyethanesulfonic acid, benzenesulfonic acid, p-toluenesulfonic acid, cyclohexanesulfamic acid, salicylic acid, p-aminosalicylic acid, 2-phenoxybenzoic acid and 2-acetoxybenzoic acid, with mesylic acid being preferred. Such salts (or similar salts) can all be prepared by conventional means. The nature of the salt is not essential so long as it is non-toxic and does not substantially interfere with the desired pharmacological activity. Conversion of 1-amino-1,3,3,5,5-pentamethylcyclohexane to a pharmaceutically acceptable salt thereof can be accomplished by mixing 1-amino-1,3,3,5,5-pentamethylcyclohexane with at least one molecular equivalent of the selected acid in the presence of an inert organic solvent using a conventional method. Isolation of the salt can be accomplished by a method known in the art, such as by causing precipitation with a nonpolar solvent that exhibits limited solubility for the salt (for example, ether).

The term "pharmaceutically acceptable" as it relates to a component (or substance, compound or agent) encompasses components (or substances, compounds or agents) that do not affect human safety and/or that are sufficiently accepted by humans after administration. Typically, when used herein, the term "pharmaceutically acceptable" means listed in the Pharmacopeia as being accepted by a regulatory agency or being generally approved, for use in mammals and more specifically humans.

The terms "polymorphism" and "polymorphism-type" include neramexane or pharmaceutically acceptable salts thereof that forms variety of crystal structures or lattices.

The term "prodrug" encompasses substances derived from neramexane or substances serving as sources for preparation of neramexane, such substances being administered in forms having little or no activity in the body compared to neramexane itself.

The term "solvate compound" encompasses substances formed by bonding of 1-amino-1,3,3,5,5-pentamethylcyclohexane with a solvent molecule or its attraction of a solvent molecule. A solvate compound where the solvent is water is known as a "hydrate".

The term "conjugant" encompasses substances formed by covalent bonding or non-covalent bonding of neramexane with a carrier.

The term "derivative" refers to neramexane having the amino group derivatized with 1 or 2 alkyl groups.

The term "isomer" refers to the possible stereoisomers of neramexane, such as conformational isomers and enantiomers or diastereomers.

The terms "approximately" and "about" usually means within 20%, or within 10%, such as within 5%, of a given value or range.

The terms "treat" and "treatment" are used herein in the sense of alleviation or reduction of at least one condition or symptom of a patient. Also the term "treat", in the sense used for the invention, means halting or delaying onset (the period before condition is clinically manifested) and/or reducing disease progression or risk of exacerbation.

A therapeutic effect can be evaluated by physician inquiry using a questionnaire form such as TRSw (Tinnitus Rating Scale One-Week Version) (Acta Otolaryngol., 2013, 133(5), 491-498).

As used herein, the term "tinnitus" encompasses all symptoms including subjective and objective tinnitus.

Tinnitus that is to be treated by the drug according to one embodiment of the invention is that associated with age-related hearing loss. The tinnitus may be occurring before onset of age-related hearing loss, or occurring simultaneously with onset of age-related hearing loss, or having occurred subsequent to (after) onset of age-related hearing loss.

As used herein, the term "age-related hearing loss" means sound perception hearing loss that progresses with age. For the purpose of the invention, it is, for example, hearing loss found to the same degree in both ears, and hearing loss progressing slowly with age.

Also as used herein, the term "age-related hearing loses" is used in the same sense as "geriatric hearing loss" and "senile hearing loss".

Also as used herein, the term "sound perception hearing loss" refers to hearing loss considered to be accompanied by organic lesion in the internal ear or at a site from the internal ear to the auditory center.

Tinnitus that is to be treated by the drug according to one embodiment of the invention is tinnitus associated with auditory disorder in the range of the human voice, at an auditory threshold value exceeding 25 dB (especially 40 dB).

Also, tinnitus that is to be treated by the drug according to one embodiment of the invention is tinnitus associated with age-related hearing loss exhibiting auditory disorder in the range of the human voice, at an auditory threshold value exceeding 25 dB (especially 40 dB).

As used herein, the term "range of the human voice" means the range in a frequency band of 500 Hz to 4000 Hz.

For the purpose of the present specification, "auditory threshold value" of "auditory disorder in the range of the human voice" can be determined by indicating the sound pressure level of the minimum pure tone that can be audibly detected in a noiseless environment, with measurement using an audiometer, for example.

Also as used herein, the term "auditory threshold value" is used in the same sense as "audible threshold value" and "threshold of audibility".

Also as used herein, the term "auditory disorder" is used in the same sense as "hearing impairment", "loss of hearing" and "hearing loss".

In one embodiment of present invention, tinnitus that is to be treated by the drug is tinnitus associated with age-related hearing loss at hearing threshold of 25dB or more in both ears in 8000Hz.

In one embodiment of present invention, tinnitus that is to be treated by the drug is tinnitus associated with age-related hearing loss at hearing threshold of 25dB or more in both ears in 4000Hz and 8000Hz.

As used herein, the term "the tinnitus includes two or more different tinnitus sounds" means that the tinnitus has tinnitus sounds with two or more different tones. It can be evaluated whether or not the tinnitus includes two or more different tinnitus sounds by, for example, physician inquiry or questionnaire to the patient.

According to a particular embodiment, a specified feature is that the number of tinnitus sounds is "two" or "three or more" according to Standard Tinnitus Examination Methods 1993 (Tinnitus Study Group).

As used herein, the term "tinnitus including a tinnitus sound that is not high-frequency alone" means tinnitus which includes a low-frequency tinnitus sound or a tinnitus sound whose pitch of sound cannot be identified, without being limited by whether or not high-frequency tinnitus sound is present. It can be evaluated whether or not the tinnitus includes a tinnitus sound that is not high-frequency alone by, for example, physician inquiry or questionnaire to the patient.

According to a particular embodiment, a specified feature is that the pitch of the tinnitus sound is "low pitch" or "neither", according to Standard Tinnitus Examination Methods 1993 (Tinnitus Study Group).

As used herein, the term "high frequency" means, for example, a frequency band of 4000Hz or more.

The drug of the invention may contain only neramexane or its pharmaceutically acceptable salt alone, or it may contain neramexane or its pharmaceutically acceptable salt together with a carrier or the like. The dosage form of the drug of the invention may be as a solid formulation, such as capsules, tablets or a similar form.

The drug of the invention can also be orally administered as a semi-solid or liquid formulation.

For a solid formulation in the form of tablets or capsules, the neramexane or its pharmaceutically acceptable salt may be combined with the following, for example: nontoxic pharmaceutically acceptable excipients such as binders (for example, gelatinized corn starch, polyvinylpyrrolidone or hydroxypropyl methyl cellulose); fillers (for example, lactose, sucrose, glucose, mannitol, sorbitol and other reducing or non-reducing sugars, microcrystalline cellulose, calcium sulfate or calcium hydrogenphosphate); lubricants (for example, magnesium stearate, talc or silica, stearic acid, sodium stearyl fumarate, glycerin behenate, calcium stearate and similar substances); disintegrators (for example, potato starch or starch sodium glycolate)'; or moistening agents (for example, sodium lauryl sulfate), coloring agents and flavoring agents, gelatin, sweetener, natural and synthetic rubber (for example, acacia, tragacanth or alginic acid), buffer salts, carboxymethyl cellulose, polyethylene glycol, waxes, and similar substances.

Tablets can be coated with a concentrated sugar solution which may contain, for example, gum arabic, gelatin, talc, titanium dioxide or similar substances. Alternatively, tablets may be coated with a polymer that dissolves in a readily volatile organic solvent or a mixture of organic solvents. According to a specific mode, neramexane is compounded into immediate release (IR) or modified release (MR) tablets. An immediate release solid dosage form allows release of most or all (for example, 90% or more) of the active ingredient in a short period of time such as 60 minutes or less, and allows rapid absorption of the drug.

A modified release solid oral drug allows sustained release of the active ingredient over a prolonged period in order to maintain a therapeutically effective blood plasma level for long periods, and/or to examine other pharmacokinetic properties of the active ingredient or other components. For example, neramexane mesylate can be formulated as a modified release dosage form (for example, a modified release tablet) for supply of a 50 mg dosage of neramexane mesylate.

For a soft gelatin capsule formulation, neramexane or its pharmaceutically acceptable salt may be compounded together with a vegetable oil or polyethylene glycol, for example. Hard gelatin capsules may contain granules of the active substance, using any of the aforementioned excipients for tablets, such as lactose, saccharose, sorbitol, mannitol, starch (for example, potato starch, corn starch or amylopectin), cellulose derivatives or gelatin. Also, a liquid or semi-solid drug may be filled into hard gelatin capsules.

Neramexane or its pharmaceutically acceptable salt may also be introduced into microspheres or microcapsules produced from polyglycolic acid/lactate acid (PGLA) (for example, see US Patent No. 5,814,844, No. 5,100,669, No. 4,849,222; International Patent Publication No. WO95/11010 and No. 93/07861). A biocompatible polymer, for example, a polylactic acid, polyglycolic acid, polylactic acid and polyglycolic acid copolymer, poly(ε-caprolactone), polyhydroxybutyric acid, poly(ortho ester), polyacetal, polyhydropyran, polycyano acrylate, or a hydrogel crosslinked or amphiphilic block copolymer, may be used to achieve modified release of the drug.

A semi-solid or liquid formulation of neramexane or its pharmaceutically acceptable salt may also be used. Neramexane may constitute 0.1 to 99 wt% of the formulation, and more specifically, 0.2 to 50 wt% for a suitable formulation that is to be administered orally.

Neramexane or its pharmaceutically acceptable salt may be administered as a modified release formulation. A modified release dosage form provides a means of improving patient compliance and ensuring effective and safe treatment, by reducing incidence of side-effects. Compared to an immediate release dosage form, a modified release dosage form has extended pharmacological action following administration and lower plasma concentration of drug over the administered period, whereby it is possible to eliminate or reduce sharp peaks.

A modified release dosage form may comprise a core, which is coated with or containing the drug. The core is then coated with a sustained-release modified polymer in which the drug is dispersed. The sustained-release modified polymer gradually disintegrates and releases the drug over time. It is thus prepared so that the outermost layer of the composition is effectively reduced, so that when the composition is exposed to an aqueous environment, i.e. in the gastrointestinal tract, the drug diffuses through the coating layer. The net diffusion rate of the drug will depend on the ability of gastric juice to penetrate the coating layer or matrix, and on the solubility of the drug itself.

According to another mode of the invention, neramexane or its pharmaceutically acceptable salt is prepared as an oral liquid formulation. A liquid formulation for oral administration may be in the form of, for example, a liquid, syrup, emulsion or suspension, or it may be provided as a dry product to be reformulated with water or another appropriate vehicle before use. A formulation for oral administration may be appropriately formulated to provide controlled or extended release of the active compound.

For oral administration of a liquid form, neramexane or its pharmaceutically acceptable salt may be combined with a nontoxic pharmaceutically acceptable inert carrier (for example, ethanol, glycerol or water), a suspending agent (for example, sorbitol syrup, a cellulose derivative or a hydrogenated edible fat), an emulsifier (for example, lecithin or acacia), a nonaqueous vehicle (for example, almond oil, an oilbased ester, ethyl alcohol or a fractionated vegetable oil), a preservative (for example, methyl or propyl-p-hydroxybenzoate or sorbic acid), and similar substances. A stabilizer such as an antioxidant (BHA, BHT, propyl gallate, sodium ascorbate or citric acid) may also be added to stabilize the dosage form. For example, a solution may contain a mixture of approximately 0.2 to approximately 20 wt% neramexane, a sugar, and ethanol, water, glycerol and propylene glycol. Optionally, such a liquid formulation may contain a coloring agent, flavoring agent, saccharin and carboxymethyl-cellulose as a thickener or other excipient.

According to another mode, a therapeutically effective dose of neramexane or its pharmaceutically acceptable salt is administered as an oral solution containing a preservative, sweetener, solubilizing agent and solvent. An oral solution may contain one or more buffers, flavoring agents or additional excipients. According to yet another mode, peppermint or other flavoring agents are added to a neramexane oral liquid formulation.

For inhaled administration, neramexane can be conveniently supplied in the form of an aerosol atomized presentation from a compressed pack or nebulizer, using an appropriate propellant such as dichlorodifluoromethane, trichlorofluoromethane, dichlorotetrafluoromethane, carbon dioxide or another suitable gas. In the case of a compressed aerosol, the administration unit can be established by providing bubbles for supply of a metered dose. Gelatin capsules and a cartridge for use in an inhalator or syringe may be included so as to contain a powder mixture of a compound and suitable powder base, such as lactose or a starch.

A solution for parenteral administration by injection may be formulated as an aqueous solution of a pharmaceutically acceptable salt of neramexane at a concentration of approximately 0.5% to approximately 10 wt%. Such a solution may also contain a stabilizer and/or buffering agent, and may be provided as an ampule for different administration units.

The drug of the invention may be directly injected by, for example, bolus injection or continuous injection, in a parenteral manner, i.e. intravenous (i.v.), intracerebroventricular (i.c.v.), subcutaneous(s.c.), intraperitoneal (i.p.), intramuscular (i.m.), intratympanic (i.t.), subdermal (s.d.) or intradermal (i.d.) administration. A formulation for injection may be provided in a unit dosage form, such as an ampule or multidose container, together with an added preservative. On the other hand, a pharmaceutically acceptable salt of neramexane may exist as a powder form for reformulation with a suitable vehicle, for example, aseptic pyrogen-free water before use.

The drug of the invention may be provided as a medical pack or kit comprising one or more containers comprising neramexane or its pharmaceutically acceptable salt and optionally the many components in the formulation. According to a specific mode, neramexane is provided as an oral solution (2 mg/ml) for administration using a 2-teaspoon syringe (dosage KORC^{R}). An individual oral syringe has lines on the right side (lowered tip) of the syringe showing teaspoon units and blue hatch marks for measurement with the lines, on the left side of the syringe showing ml units.

The optimal therapeutically effective dose can be determined in consideration of the form of administration of the drug, the patient (for example, body weight, health, age and gender), and the preference and experience of the acting physician.

The dosing unit for rectal administration may be prepared in the form of a solution or suspension, or a suppository or retention enema containing neramexane mixed with a triglyceride base, or a gelatin rectal capsule containing neramexane mixed with a vegetable oil or paraffin oil.

The toxicity and therapeutic effect of neramexane or its pharmaceutically acceptable salt may be measured, for example, by determining the LD₅₀ (50% lethal dose for a group) and ED₅₀ (50% therapeutically effective dose for a group), in a standard treatment method for experimental animals. The dose ratio between the therapeutic effect and toxicity effect is the therapeutic index, and it can be expressed as the ratio LD₅₀/ED₅₀. Neramexane or its pharmaceutically acceptable salt/composition preferably has a high therapeutic index.

An appropriate daily dose for neramexane or its pharmaceutically acceptable salt for human treatment is approximately 0.01 to 10 mg/kg body weight for oral administration and 0.001 to 10 mg/kg body weight for parenteral administration. For example, a suitable daily dosage of neramexane mesylate for adults may be a dose of 25 mg, 50 mg or 75 mg. An equimolar amount of a separate pharmaceutically acceptable salt, solvate compound, isomer, conjugant, prodrug, polymorphism or derivative, such as neramexane hydrochloride, is also suitable.

Generally, neramexane or its pharmaceutically acceptable salt, such as neramexane mesylate is administered in a range of about 5 mg to about 150 mg/day, about 5 mg to about 100 mg/day or about 5 mg to about 75 mg/day, or at about 50 mg/day or about 75 mg/day.

The daily doses indicated throughout the present specification may be administered, for example, once, twice or three times a day, as single or double dose units.

The treatment period may be a short period such as several weeks (for example, 8 to 16 weeks), or it may be a long period until the attending physician judges that no further treatment is necessary.

The drug of the invention may be administered in a form with a dose-setting scheme (titration scheme). The term "dose-setting scheme" means a treatment method such as carried out in the examples of the present specification. The patient is initially administered a low dose of neramexane or its pharmaceutically acceptable salt, and is then given the same dose or a higher dose during the treatment period.

### [Examples]

The invention will now be further explained by examples, with the understanding that the scope of the invention is not limited by the examples.

Film coated tablets comprising 12.5 mg neramexane mesylate were produced by the method disclosed in International Patent Publication No. WO2009/033649.

The efficacy of neramexane mesylate on patients with tinnitus associated with age-related hearing loss, tinnitus associated with auditory disorder in the range of the human voice at an auditory threshold value exceeding 25 dB, tinnitus that includes two or more different tinnitus sounds or tinnitus that includes a tinnitus sound that is not high-frequency alone, was evaluated in a 24-week, placebo controlled, randomized, double-blind comparative trial.

The film coated tablets containing 12.5 mg neramexane mesylate or placebo tablets were administered for 24 weeks to patients having tinnitus. The dosage was started from 12.5 mg/day, gradually increasing the dose to 50 mg/day over 4 weeks, and maintaining the same dose thereafter.

The therapeutic effect of neramexane mesylate for the patients was evaluated using the TRSw score. The evaluation results are shown in Table 1. Statistical analysis was performed using a mixed-effect model repeated measure (MMRM) taking into consideration the covariance structure of error for the patient, with the change in TRSw score from before administration of neramexane mesylate tablets or placebo tablets (0 weeks) to each evaluation time point as the response variable, and with the drug-administered group, evaluation time point, interaction between drug-administered group and evaluation time point, 0-week TRSw score, gender and body weight as anchor effects. The numerical values in the table represent least mean squares for the change in TRSw score at 24 weeks after administration. A change in score of less than 0 indicates a higher therapeutic effect.

[Table 1]

**Table 1 Therapeutic effect of neramexane mesylate**

| Test group | Change in TRSw score | | |
|---|---|---|---|
| | Neramexane | Placebo | Neramexane - placebo |
| Tinnitus associated with age-related hearing loss | -2.5 | -2.0 | -0.5 |
| Tinnitus associated with auditory disorder (>25 dB) in the range of the human voice | -1.7 | -1.1 | -0.6 |
| Tinnitus associated with age-related hearing loss, exhibiting auditory disorder (>25 dB) in the range of the human voice | -3.6 | -1.6 | -2.0 |
| Tinnitus associated with age-related hearing loss at hearing threshold of 25dB or more in both ears in 8000Hz. | -2.2 | -0.7 | -1.5 |
| Tinnitus associated with age-related hearing loss at hearing threshold of 25dB or more in both ears in 4000Hz and 8000Hz. | -2.5 | -0.5 | -2.0 |
| Tinnitus including two or more different tinnitus sounds | -2.2 | -1.7 | -0.5 |
| Tinnitus including two or more different tinnitus sounds, associated with age-related hearing loss | -4.1 | -1.4 | -2.7 |
| Tinnitus including two or more different tinnitus sounds, associated with age-related hearing loss, exhibiting auditory disorder (>25 dB) in the range of the human voice | -5.0 | -0.3 | -4.7 |
| Tinnitus including tinnitus sound not of high-frequency alone | -2.0 | -1.3 | -0.7 |

Compared to placebo, neramexane mesylate exhibited a superior therapeutic effect for patients affected by tinnitus associated with age-related hearing loss, patients affected by tinnitus with auditory disorder in the range of the human voice at an auditory threshold value exceeding 25 dB, patients affected by tinnitus including two or more different tinnitus sounds, and patients affected by tinnitus including a tinnitus sound that is not high-frequency alone. Also, surprisingly, neramexane mesylate exhibited an unexpectedly excellent therapeutic effect for patients affected by tinnitus associated with age-related hearing loss and exhibiting auditory disorder in the range of the human voice at an auditory threshold value exceeding 25 dB, patients affected by tinnitus including two or more different tinnitus sounds associated with age-related hearing loss, and patients affected by tinnitus including two or more different tinnitus sounds associated with age-related hearing loss and exhibiting auditory disorder in the range of the human voice at an auditory threshold value exceeding 25 dB. Thus, it was found that neramexane mesylate is a very effective treatment agent for tinnitus, for patients with tinnitus associated with age-related hearing loss and exhibiting auditory disorder in the range of the human voice at an auditory threshold value exceeding 25 dB, patients with tinnitus including two or more different tinnitus sounds associated with age-related hearing loss, and patients with tinnitus including two or more different tinnitus sounds associated with age-related hearing loss and exhibiting auditory disorder in the range of the human voice at an auditory threshold value exceeding 25 dB.

## Claims

1. A drug for treatment of patients having tinnitus associated with age-related hearing loss, comprising neramexane or a pharmaceutically acceptable salt thereof.

2. A drug for treatment of patients having tinnitus associated with auditory disorder in the range of the human voice at an auditory threshold value exceeding 25 dB, comprising neramexane or a pharmaceutically acceptable salt thereof.

3. A drug for treatment of patients having tinnitus associated with age-related hearing loss, comprising neramexane or a pharmaceutically acceptable salt thereof, wherein the age-related hearing loss exhibits auditory disorder in the range of the human voice at an auditory threshold value exceeding 25 dB.

4. A drug for treatment of patients having tinnitus, comprising neramexane or a pharmaceutically acceptable salt thereof, wherein the tinnitus includes two or more different tinnitus sounds.

5. A drug for treatment of patients having tinnitus associated with age-related hearing loss, comprising neramexane or a pharmaceutically acceptable salt thereof, wherein the tinnitus includes two or more different tinnitus sounds.

6. A drug for treatment of patients having tinnitus associated with age-related hearing loss, comprising neramexane or a pharmaceutically acceptable salt thereof, wherein the age-related hearing loss exhibits auditory disorder in the range of the human voice at an auditory threshold value exceeding 25 dB, and wherein the tinnitus includes two or more different tinnitus sounds.

7. A drug for treatment of patients having tinnitus, comprising neramexane or a pharmaceutically acceptable salt thereof, wherein the tinnitus sound is not high-frequency alone.

8. A drug for treatment of patients having tinnitus associated with age-related hearing loss, comprising neramexane or a pharmaceutically acceptable salt thereof, wherein the age-related hearing loss is at hearing threshold of 25dB or more in both ears in 8000Hz.

9. A drug for treatment.of patients having tinnitus associated with age-related hearing loss, comprising neramexane or a pharmaceutically acceptable salt thereof, wherein the age-related hearing loss is at hearing threshold of 25dB or more in both ears in 4000Hz and 8000Hz.
